# EUROPEAN PATENT SPECIFICATION

(11) **EP 1 495 137 B1**
(45) Date of publication and mention of the grant of the patent: **14.09.2005**
(21) Application number: 03710595.4
(22) Date of filing: 04.04.2003
(51) Int. Cl.: C12Q 1/68

(54) **PRIMER EXTENSION BASED METHOD EMPLOYING NUCLEOTIDES LABELLED VIA CLEAVABLE LINKERS**
AUF PRIMER VERLÄNGERUNG BASIERENDES VERFAHREN, DAS NUKLEOTIDE VERWENDET, DIE ÜBER SPALTBARE LINKER MARKIERT SIND
PROCEDE BASE SUR L'EXTENSION D'AMORCE ET EMPLOYANT DES NUCLEOTIDES MARQUES VIA UN ELEMENT DE LIAISON CLIVABLE

(30) Priority: 04.04.2002 SE 0201024; 04.04.2002 US 369599 P
(43) Date of publication of application: 12.01.2005
(73) Proprietor: Biotage AB, 753 18 Uppsala (SE)
(72) Inventor: OLSSON, Charlotta, S-753 27 Uppsala (SE); TOOKE, Nigel, S-741 44 Knivsta (SE)
(74) Representative: Bergstrand, Mikael Gudmundsson
(86) International application number: PCT/SE2003/000547
(87) International publication number: WO 2003/085135

(56) References cited:
- WO-A1-00/50642
- WO-A1-98/44152
- WO-A2-00/53812
- WO-A2-01/11083
- WO-A2-01/94546
- US-A- 5 268 486

## Description

The present invention relates to the field of sequence determination of polynucleotides, and specifically to a sequencing-by-synthesis method in which a fluorescently activated nucleotide is detected after incorporation into a primer-template complex and deactivated after detection, where the detectable group is linked to the dNTP with a disulfide-containing linkage. The present invention also provides a kit for carrying out the above method.

### BACKGROUND

Methods for DNA sequencing have been of great importance for most genomic analysis during the last decades. These methods are of great value within the life sciences and have made possible the great advances seen within the field of biotechnology. Not only have these techniques allowed sequencing of short polynucleotides, but also of entire genes and other genetic material. Two different approaches have traditionally been used. The first is a chemical degradation method, which was developed by Maxam and Gilbert (Proc. Natl. Acad. Sci., 74, 560, (1977)). The second approach was developed by Sanger et al, (Proc. Natl. Acad. Sci., 74, 5463, (1977)) and is based on an enzymatic extension of a primer bound to a template.

The enzymatic approach utilizes dideoxynucleotides to generate the sequencing fragments, this is also known as the dideoxy sequencing method.

The above-mentioned methods have a number of drawbacks. They require a means for separating the generated fragments, based on size. This separation is performed on a polyacrylamide gel, and is time-consuming, uses large amounts of expensive chemicals and is severely limits the number of bases that can be separated in one experiment. The reading of the gels is also time-consuming. Furthermore, the fact that certain fragments can form different structures within the gel causes the mobility of these fragments to change.

A number of improvements to these methods have been made in recent years, in order to increase efficiency and speed. Reagents for running the sequencing reactions have been improved, automatic sequencing-machines have been developed for reading the gels, different labels for incorporation into nucleotides have been developed, and so on. The use of different dyes to label the nucleotides has alleviated the need for running the products of the four different sequencing reaction products in different lanes on a gel.

Despite these developments, there is a need for cheaper and faster sequencing methods. A number of such methods have been developed or proposed. These methods include sequencing by hybridization (R. Drmanac et al, Genomics, 4, (1989) 114; Drmanac et al, DNA Cell Biology, 9, (1990), 527; Bains et al, J. Theor. Biol., 135, (1988), 303-307, among other references), fluorescent detection of single molecules (Jett et al, J. Biomol. Struct. Dyn., 7, (1989), 301; Nguyen et al, Anal. Chem., 56, (1987), 348, or sequencing by scanning tunnel microscopy. The above-mentioned methods have different drawbacks, and are in general cost-inefficient and/or not feasible.

WO 01/94546 relates to a method for detecting and analyzing point mutations and SNPs by performing a single base pair extension. Thirty to ninetyfive percent of the nucleotides in the extension step can be labelled.

WO 01/11083 describes a method for detecting sequence deletions, which method involves a step where a nucleic acid molecule is extended by a adding a single labeled nucleotide. A mixture of labelled and unlabelled nucleotides is used in said step.

WO 98/44152 discloses a method for sequencing different nucleic acid molecules present at different locations in parallel. The method involves primer extension using labeled nucleotides. Mixtures of labeled/natural nucleotides containing labeled nucleotides of <50 %, <20 % or <10 % of the total are mentioned. The reason for using such mixtures is to reduce costs and also possibly to reduce quenching effects if certain labels are used where signals from such labels interfere with each other. There is no attempt to remove the signal incorporated. Rather, the signal accumulates for each additional incorporation. The fluorescence variant of this method cannot be expected to work for longer sequences since the sensitivity will be successively reduced as the total signal increases.

A different method, the so-called sequencing-by-synthesis method was first described by Melamede, US 4863849. In short, the method can be described as follows; 1) an activated nucleotide triphosphate is added to a primer-template complex; 2) the activated nucleotide is detected; 3) step 1) is repeated, whereupon the sequence can be deduced from positive incorporation of nucleotides. In this general description, the activated group can be located anywhere on the dNTP molecule; in US 5,302,509, the activated group is attached to the sugar moiety at the 3'-position, whereas in WO 93/21340, the activated group is attached to the base. Nyrén discloses a third strategy in WO 98/13523 and WO98/28440 in which the activation is related to the detection of released pyrophosphate during the primer extension step.

WO 00/53812 and WO 00/50642 describe the use of a nucleotide where a disulfide-containing linker is used for coupling a dye to the nucleotide. This enables easy removal of the dye by redox cycling. In WO 00/53812 the dye is linked to the base (only dCTP is described) and in WO 00/50642 the dye is attached to the 3'- position of the sugar moiety.

One of the advantages with a sequencing-by-synthesis method utilizing a dye labeled nucleotide is that a localized signal is obtained. This means that applications such as sequence determination of polynucleotides on an array, where all polynucleotides may or may not be different is possible. Furthermore, only one enzyme is needed, which makes the sequence determination cheaper.

The present invention aims at providing an improved sequencing-by-synthesis method which is cheaper and shows an increased accuracy and sensitivity compared to state-of-the-art methods.

### SUMMARY OF THE INVENTION

In one aspect, the invention relates to a method for determining the sequence of a nucleic acid molecule comprising the steps of;
a) providing a single-stranded form of said nucleic acid molecule;
b) hybridizing a primer to said single stranded form of said nucleic acid molecule to form a template/primer complex;
c) enzymatically extending the primer by the addition of a polymerase and a mixture of at least one nucleotide and at least one labeled derivative of the at least one nucleotide, wherein the at least one labeled derivative of the at least one nucleotide comprises a label linked to the nucleotide via a cleavable link and wherein the amount of labeled derivative of the at least one nucleotide in said mixture of the at least one nucleotide and the labeled derivative of the at least one nucleotide is within the range of 1-50 mole-%, 1-40 mole-%, 1-30 mole-%, or 1-20 mole-%, preferably in the range of 5-50 mole-%, 5-40 mole-%, 5-30 mole-%, or 5-20 mole-%, or more preferably in the range of 10-50 mole-%, 10-40 mole-%, 10-30 mole-%, or 10-20 mole-%.
d) determining the type of nucleotide added to the primer;
wherein, steps c) to d) above are repeated at least once.

In a further embodiment, the label is neutralized after step d) by the addition of a label-interacting agent or by bleaching, preferably by photo-bleaching. The label can be neutralized by bleaching (photo bleaching) or by adding a compound that neutralizes the emitted fluorescence, such as another label, then reducing the emitted light by quenching.

In certain embodiments it is preferable to cleave off the label from the nucleotide. This is made possible by using a linker between the nucleotide and label that is cleavable by e.g. a reducing agent. Thus, a method according to the above is provided, in which the link between the incorporated nucleotide and the label is cleaved after step d). According to this, a method according to the above is provided, in which the link between the fluorophore and nucleotide is an S-S bridge.

In one embodiment the cleavage is performed by the addition of a reducing agent, thereby exposing a thiol group.

In one embodiment, the exposed thiol group is capped by a suitable reagent, such as iodoacetamide or N-ethylmaleimide.

The object of the invention may be met by using a linker that is short enough to prevent interaction between adjacent labels. According to this, the length of the linker between the disulfide bridge and the base of the nucleotide is preferably shorter than 8 atoms. Thus, in a further embodiment, the linker between the disulfide bridge and the base is shorter than 8 atoms.

In one embodiment step c) is performed at a pH below 7, preferably at a pH below 6.5, or more preferably at a pH below 6.

In a further embodiment, the derivative of said nucleotide is a dideoxynucleotide or an acyclic nucleotide analog.

In yet a further embodiment, an agent chosen from the group comprising the following; alkaline phosphatase, PPi-ase, apyrase, dimethylsulfoxide, polyethylene glycol, polyvinylpyrollidone, spermidine, detergents such as NP-40, Tween 20 and Triton X-100; various proteins that affect secondary structure of DNA including Single Stranded DNA Binding Protein (SSB) or the protein of Gene 32, is added.

A mixture of at least one nucleotide and a labelled derivative of the at least one nucleotide is used, wherein the at least one labeled derivative of the at least one nucleotide comprises a label linked to the nucleotide via a cleavable link and wherein the amount of labeled derivative of the at least one nucleotide in said mixture of the at least one nucleotide and the labeled derivative of the at least one nucleotide is within the range of 1-50 mole-%, 1-40 mole-%, 1-30 mole-%, or 1-20 mole-%, preferably in the range of 5-50 mole-%, 5-40 mole-%, 5-30 mole-% or 5-20 mole-%, and even more preferably in the range of 10-50 mole-%, 10-40 mole-%, 10-30 mole-% or 10-50 mole-%.

A further aspect of the invention is a kit which comprises, in separate compartments, a mixture according to previously mentioned aspects, and a reducing agent and optionally at least one of the following components; a DNA polymerase, a reducing agent, a carrier; a capping agent, an apyrase, an alkaline phosphatase, a PPi-ase, a single strand binding protein or the protein of Gene 32, for performing the method according to any of the steps in the above-mentioned methods.

Thus, a mixture of a (unlabeled) nucleoside triphosphate and a derivative of said nucleoside triphosphate is used, wherein said derivative of said nucleoside triphosphate comprises a disulfide bridge as part of a link between a label and the nucleoside triphosphate. Accordingly, a mixture of at least one nucleoside triphosphate and at least one labeled derivative of the at least one nucleoside triphosphate is used, wherein the at least one labeled derivative of the at least one nucleoside triphosphate comprises a disulfide bridge between the label and the nucleoside triphosphate, wherein the amount of labeled derivative of the at least one nucleoside triphosphate in said mixture of the at least one nucleoside triphosphate and the labeled derivative of the at least one nucleoside triphosphate is within the range of 1-50 mole-%, 1-40 mole-%, 1-30 mole-%, 1-20 mole-%. A preferred amount is within the range of 5-50 mole-%, 5-40 mole-%, 5-30 mole-% or 5-20 mole-%, and an even more preferred amount is within the range of 10-50 mole-%, 10-40 mole-%, 10-30 mole-% or 10-20 mole-%.

### DEFINITIONS

The term "oligonucleotide" as used herein, includes linear oligomers of natural or modified monomers or linkages, including deoxyribonucleosides, ribonucleosides, and the like, capable of specifically binding to a polynucleotide by way of a regular pattern of monomer-to-monomer interactions, such as Watson-Crick type of base pairing, base-stacking, Hoogsteen or reverse Hoogsteen types of base-pairing, or the like. Usually monomers are linked by phosphodiester bonds or analogs thereof to form oligonucleotides ranging in size from a few monomeric units, e.g. 3-4, to several tens of monomeric units, e.g. 40-60. Whenever an oligonucleotide is represented by a sequence of letters, such as "ATGCCTGG", it will be understood that the nucleotides are in 5'-> 3'order from left to right and that "A" denotes deoxyadenosine, "C" denotes deoxycytosine, "G" denotes deoxyguanosine and "T" denotes deoxythymidine.

The term "primer" as used herein is meant to mean an oligonucleotide, which is used in extension reactions and accepted by a DNA polymerase, i.e. the primer has a 3'-OH-group. The primer is annealed or hybridized to a template to form a primer/template complex ready for an extension reaction.

As defined herein, the term "nucleotide" is used to represent DNA or RNA building blocks, such as deoxynucleotides or ribonucleotides. A nucleotide will also represent deoxy- or ribonucleoside triphosphates and modified representatives of the building blocks.

As used herein, the term "nucleosides" includes the natural nucleosides, including 2'-deoxy and 2'-hydroxyl forms, as described in Kornberg and Baker, DNA replication, 2^{nd} Ed. (Freeman, San Francisco. 1992). "Analogs" in reference to nucleosides includes synthetic nucleosides having modified base moieties and/or modified sugar moieties, e.g. described by Scheit, Nucleotide Analogs (John Wiley, New York, 1980); Uhlman and Peyman, Chemical Reviews, 90: 543-584 (1990), or the like, with the only proviso that they are capable of specific hybridization. Such analogs include synthetic nucleosides designed to enhance binding properties, reduce complexity, increase specificity, reduce secondary structures in polynucleotides, and the like, such as LNA and PNA

As used herein, the term "sequence determination" or "determining a nucleotide sequence" in reference to polynucleotides includes determination of partial as well as full sequence information of the polynucleotide. That is, the term includes sequence comparisons, fingerprinting, and similar types of information of a target polynucleotide, as well as identification and ordering of nucleosides, usually each nucleoside in a polynucleotide. The term also includes the determination or the identification of one, two, or three of the four types of nucleotides within a polynucleotide.

As defined herein, the term "template" is defined as the DNA chain that provides directions for the sequence of nucleotides, where each added nucleotide is selected by base-pair matching.

As defined herein, the term "label" is meant a molecule, which is possible to detect in a suitable manner. In particular, the terms "dye", "label" or "dye-label" include fluorescent molecules such as fluorescein, cyanine dyes, like Cy-3, Cy-5, Cy-7, Cy-9 disclosed in U.S. 5, 268,486 (Waggoner et al.) or variants thereof, such as Cy3.5 and Cy5.5, but may also include molecules such as Rhodamine, BODIPY, ROX, TAMRA, R110, R6G, Joe, HEX, TET, Alexa or Texas Red.

As defined herein, the term "labeled nucleotide" or "dye-labeled nucleotide" means a nucleotide, which is connected to a label or dye-label as defined above.

As used herein, the term "array" refers to a heterogeneous pool of nucleic acid molecules that is distributed over a support matrix. These molecules, differing in sequence, are spaced at a distance from one another sufficient to permit the identification of discrete features of the array. It may also refer to miniaturized surfaces comprising ordered immobilized oligonucleotides, DNA or RNA molecules.

As defined herein, the term "carrier" is used to represent any support for attracting, holding or binding a polynucleotide used within the fields of biotechnology or medicine. A carrier can be a carrier, such as a gel, a bead (microparticles), a surface or a fiber. Different examples of gels are acrylamide or agarose; examples of beads are solid beads, which can contain a label or a magnetic compound; beads can also be porous, such as Sepharose beads; a surface can be the surface of glass, a plastic polymer, silica or a ceramic material - these surfaces can be used to prepare so-called "arrays". A fiber can be a starch fiber or an optical fiber and even the end of a fiber.

As defined herein, the term "capping" refers to the addition of a protecting group to an exposed part of a link between a label and a nucleotide, such as the attachment of iodoacetamide or N-ethylmaleimide to an exposed thiol group.

Buffers used in the examples are defined in the text where they are first described.

As defined herein, the term "homopolymer" or "homopolymeric stretches" is defined as at least two nucleotide residues of the same base after each other, i.e. AAₙ, TTₙ, CCₙ or GGₙ, where n>=1.

Throughout this specification and the claims, the words "comprises" and "comprising" are used in a non-exclusive sense.

It is to be understood that the terminology used herein is for the purpose of describing particular embodiments and aspects of the invention only, and is not intended to limit the scope of the invention.

It must be noted that, as used herein and in the appended claims, the singular forms "a", "an" and "the" include plural reference unless the context clearly dictates otherwise.

Unless defined otherwise, all technical and scientific terms used herein have the same meanings as commonly understood by one of ordinary skill in the art to which this invention belongs.

### BRIEF DESCRIPTION OF THE DRAWINGS

Figure 1. Effect of incubation with Iodoacetamide (IAN) on fluorescence of 2nd Cy5-SS-dCTP incorporation. Two Cy5-SS-dCTPs were incorporated in two different positions (C1 and C2) in two oligonucleotides 10b (C TGAA C) and 22b (C T C). The first incorporation was followed by treatment with buffer only (control), 1 mM IAN or 10 mM IAN.
Figure 2. Quenching effect using 100% Cy5-SS-dCTP or Cy5-dCTP. The fluorescent signal is plotted as a function of the number of bases incorporated in a homopolymer stretch.
Figure 3. Fluorescent signal after incorporating one to five Cy5-SS-dCTPs in a homopolymer stretch using 20% Cy5-SS-dCTP and 80% natural nucleotides in the reaction mix. The ratio between the fluorescent signal from Cy5 on the nucleotide and Fluorescein on the primer is plotted as a function of the number of bases incorporated in the homopolymer stretch.
Figure 4. Sequencing of the oligonucleotide E3PN19b using 50% Cy5-SS-dUTP, 20% Cy5-SS-dCTP, 30% Cy5-SS-dGTP, and 30% Cy5-SS-dATP with a final nucleotide concentration of 1 µM. The ratio between the fluorescent signal from Cy5 on the nucleotide and Fluorescein on the primer is plotted for each incorporation.
Figure 5. Selectivity curves for Cy5-SS-dCTP.The ratio between the fluorescent signal from Cy5 on the nucleotide and Fluorescein on the primer is plotted as a function of the different percentages of Cy5-SS-dCTPs in the reaction mixes.
Figure 6. Selectivity curves for Cy5-SS-dGTP.The ratio between the fluorescent signal from Cy5 on the nucleotide and Fluorescein on the primer is plotted as a function of the different percentages of Cy5-SS-dGTPs in the reaction mixes.
Figure 7. Selectivity curves for Cy5-SS-dATP.The ratio between the fluorescent signal from Cy5 on the nucleotide and Fluorescein on the primer is plotted as a function of the different percentages of Cy5-SS-dATPs in the reaction mixes.
Figure 8. Selectivity curves for Cy5-SS-dUTP.The ratio between the fluorescent signal from Cy5 on the nucleotide and Fluorescein on the primer is plotted as a function of the different percentages of Cy5-SS-dUTPs in the reaction mixes.

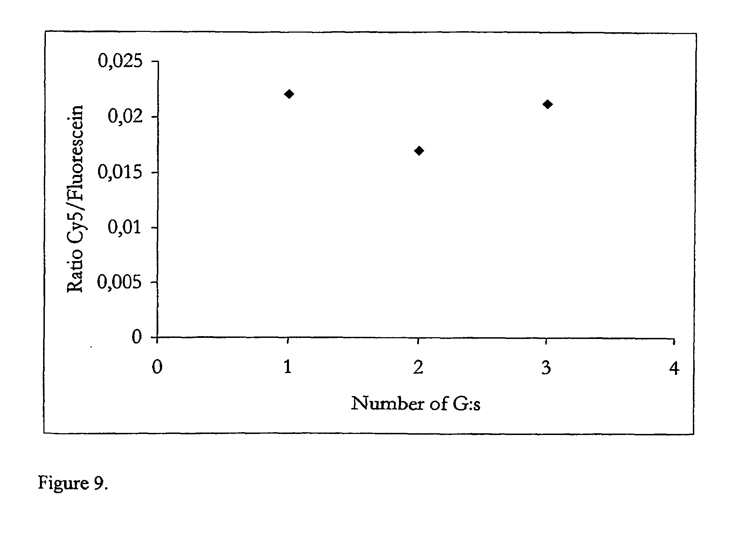

Figure 9. Fluorescent signal from incorporating one to three Cy5-SS-dGTPs in a homopolymer stretch according to WO 00/53812 using 0.2µM Cy-SS-dGTP and 0.1 µM dGTP together with 6.5U Sequenase version 2.0 (USB).

### DETAILED DESCRIPTION OF THE INVENTION

The present invention overcomes the problem with the previously mentioned sequencing-by-synthesis method and makes it feasible to perform a sequence determination without the drawbacks encountered in other methods. During attempts to reproduce the sequencing-by-synthesis method according to WO 00/53812, using 67% labeled dCTP, it was found that the relationship between the number of incorporated dye-labeled dCTPs and the signal was not linear in the range 1-3 bases. This is contrary to what one would expect, as a higher number of incorporated, labeled nucleotides should give a higher fluorescence. In addition, the incorporation of a labeled dNTP close to a dye-labeled and cleaved dNTP resulted in a signal from the second incorporation that was much lower than expected. The inventors were able to show that by lowering the ratio between labeled and non-labeled nucleotides, it was possible to obtain a signal that would increase as the number of labeled nucleotides increased. There appears to be two different ways of explaining the above failure, namely quenching or a disulfide chemistry problem.

The quenching problem can be described as follows; When two dye-labeled molecules (such as fluorophores) are located in close proximity, these molecules will interfere and reduce the emitted light. This phenomenon is called quenching. In all naturally occurring nucleic acids, the frequency of "homopolymeric stretches" is high. A homopolymeric stretch such as these would cause the signal to be quenched because it would place two fluorophores within a short distance from each other. A major problem with the method disclosed in WO 00/53812 is thus that it is difficult to reproduce when it comes to determining the sequence of polynucleotides that contain homopolymeric stretches.

### The disulfide problem can be described as follows:

The method relies on the formation and breaking of S-S bonds during the removal of fluorophore. Consider the following series of incorporations and cleavage reactions where C (or A, G, U, T) indicate a non-modified deoxynucleoside triphosphate (and N indicates any of these bases), C^{-SS-label} (or A^{-SS-label}, G^{-SS-label}, U^{-SS-label}) indicates a modified deoxynucleoside triphosphate (label-SS-dCTP) and C^{-SH} (or A^{-SH}, G^{-SH}, U^{-SH}) indicates a modified deoxynucleoside that has been cleaved with a reducing reagent, yielding HS-dCp (or HS-dAp, HS-dGp, HS-dUp)).

### First incorporation:

Primer NNNNNN-pC-SS-label
Template NNNNNN--Gp- Tp -Cp -Tp -Gp - NNNNNNNNNNNNNN

### First cleavage:

Primer    NNNNNN-pC^-SH
Template  NNNNNN -Gp- Tp -Cp -Tp -Gp - NNNNNNNNNNNNN

### Second incorporation:

Primer NNNNNN-pC^-SH-pA^-SS-label
Template NNNNNN--Gp- -Tp -Cp -Tp -Gp - NNNNNNNNNNNNN

At this point the following reaction may occur on the extended primer:

S----S
|      |
NNNNNN-pC-SH - pA-SS-label -> NNNNNN - pC - pA + label-SH

This means that the thiol group on the incorporated base reacts with the disulfide bridge of the newly-incorporated base thus displacing the label-SH group. It is evident that such a reaction would not result in any detection of the newly incorporated base and hence makes the sequencing of the template impossible. If the linker arms attached to the bases are sufficiently long and permit the two reactive groups to interact, this reaction may even occur in cases where there are one or more unmodified bases between the base carrying the thiol group and the base carrying the fluorophore.

Having realized that the underlying reason why the previously described method does not work, i.e. that intramolecular thiol bridges are formed, the inventors have identified a number of different approaches to circumvent the above mentioned problems. Basically, the invention involves measures of reducing the probability of forming such thiol bridges. This may be accomplished through one or more of the following approaches:
1. Mixing at least one deoxynucleoside triphosphate with at least one labeled derivative of the at least one deoxynucleoside triphosphate in order to reduce the probability that two neighboring incorporated bases are modified. This is clearly a balance between incorporating sufficient fluorescent deoxynucleoside triphosphate derivative to ensure detection, and minimizing the risk of cross-reactions as described above. This is the preferred approach and has enabled the method to work. The amount of labeled derivative of the at least one deoxynucleoside triphosphate in said mixture of the at least one deoxynucleoside triphosphate and the labeled derivative of the at least one deoxynucleoside triphosphate should lie in the range of 1-50 mole-%, 1-40 mole-%, 1-30 mole-%, or 1-20 mole-%, preferably in the range of 5-50 mole-%, 5-40 mole-%, 5-30 mole-% or 5-20 mole-%, or even more preferably in the range of 10-50 mole-%, 10-40 mole-%, 10-30 mole-% or 10-20 mole-%. This amount will ensure that the level of incorporation is low enough to avoid the problems associated with quenching and intra-molecular thiol group formation, and high enough to allow for efficient detection. The level of incorporation of labeled nucleotides should be in the range of 0.1-30%, or preferably 0.1-20%, or even more preferably 1-20%. It should be noted that the level of labeled deoxynucleoside triphosphate in the reaction mixture may be higher or lower than the level of incorporation of non-modified deoxynucleoside triphosphate that is required depending on the relative selectivity of the DNA polymerase for the two forms. This approach also has additional benefits relating to the detection of homopolymer stretches (see below).
2. Simply sequencing individual template molecules or groups of molecules with only one modified deoxynucleoside triphosphate, whilst the remaining three are non-modified and added separately. This approach may, however, complicate the interpretation of so-called homopolymer stretches (see below).
3. Blocking the exposed thiol group by capping with a reagent with high reactivity towards thiol groups. Examples of such reagents include iodoacetamide and N-ethylmaleimide.
4. Reducing the length of the linker arm, i.e. reduce the number of atoms between the disulfide bridge and the nucleotide, thus reducing the possibility that the reactive groups come sufficiently close to one another for a reaction to occur.
5. Reducing the density of primer/template complexes on the carrier (by reducing the number of molecules or by increasing their distance from the carrier through linker arms) in order to minimize the possibility of intermolecular reactions.
6. The reactive species of the thiol group is the thiolate ion, -S⁻. The equilibrium reaction: -SH <=> -S⁻ has a pKₐ of approximately 8, meaning that a decrease in the pH of the environment should reduce the risk of cross-reactivity as described above. This would necessitate running the incorporation reaction, subsequent washing steps and detection at a pH below 7, or preferably below 6.5, or more preferably below 6.0.
7. It is also possible to modify the labeled nucleoside triphosphate in such a way that it also terminates further extension of the primer and thus eliminates the risk of intra-molecular reactions, which may be done by using dideoxynucleotides. Clearly, this would lead to a continuous reduction in signal during extended sequencing, which may or may not be significant depending on the level of incorporation of modified nucleosides.
8. These problems could also be solved by periodically cleaving off the label group as soon as the signal-to-noise ratio becomes poor. This would result in a stepwise increase in signal as the incorporations proceed, without the risk of forming intra-molecular thiol interactions. Cleavage could be immediately followed by capping the thiol groups with a suitable reagent, such as iodoacetamide or N-ethylmaleimide.

The inventors can convincingly show that it is possible to obtain a linear increase together with an increasing number of incorporated, labeled nucleotides, by optimizing the ratio between labeled and non-labeled nucleotides.

Quenching, and other interference between neighboring labeled molecules can be controlled by ensuring an incorporation rate of the labeled deoxynucleoside triphosphate that is sufficiently low to reduce the probability that neighboring molecules on a single extended DNA strand are indeed modified and can interact. The process that steers this incorporation is the 'selectivity' of the DNA polymerase for the different forms of the deoxynucleoside triphosphate. In other words this process relates to differences in the kinetics of the reactions for the incorporation of labeled as opposed to non-labeled deoxynucleoside triphosphate. It is most commonly observed that the kinetics for incorporation of non-labeled (natural) deoxynucleoside triphosphates is faster than that of modified deoxynucleoside triphosphates, presumably due to the ability of the enzymes active site to preferentially bind natural deoxynucleoside triphosphates. Modifications to the active site of the enzyme by introducing mutations may improve the kinetics for incorporation of modified deoxynucleoside triphosphates. The optimal ratio between labeled and non-labeled deoxynucleoside triphosphates in the reaction mix thus depends on a number of factors including the following:
1. The nature of the polymerase and its active site (including modifications to the active site)
2. The type of deoxynucleoside triphosphate - different bases affect the selectivity to different extents.
3. The nature of the fluorophore.
4. The total concentration of labeled and non-labeled deoxynucleoside triphosphates - lower total concentrations appear to reduce the selectivity of the DNA polymerase, depending on the polymerase used.
5. Other variations in the reaction mix or the immediate environment in which the polymerase operates e.g. the nature of the carrier on which the template/primer complex is immobilized.

The present invention is thus based on a sequencing method, which comprises the steps of;
1) providing a single stranded template, comprising the polynucleotide to be sequenced;
2) forming a primer/template complex by hybridizing a primer to the template;
3) extending the primer by the addition of a single labeled nucleotide, with the help of a polymerase, such as a DNA or RNA polymerase;
4) determining the type of labeled nucleotide, alternatively, detecting the incorporation of a labeled nucleotide;
5) removing or neutralizing the label;
6) repeating the steps 3-5 sequentially, thereby determining the order of nucleotides.

Reagents for the following reactions are either commercially available, or could be prepared in the laboratory of a person skilled in the art.

The DNA polymerase should lack 3'-5' exonuclease activity and accept deoxynucleotides that have been modified with fluorescent molecules. Examples include Klenow exo- (Stratagene), Sequenase, Thermo Sequenase, Thermo Sequenase II (Amersham Biosciences), rTth DNA polymerase (Applied Biosystems), Tli DNA polymerase exo- (Vent (exo-) DNA Polymerase, New England Biolabs), Deep Vent (exo-) DNA Polymerase (New England Biolabs), AmpliTaq DNA polymerase (Amersham Biosciences), Bst DNA Polymerase (New England Biolabs). DyNAzyme I and II DNA Polymerases (Finnzymes Oy).

A number of agents or additives may be used in the disclosed method for improving the performance of the sequencing reaction. Examples of such additives include the following: dimethylsulfoxide, polyethylene glycol, polyvinylpyrollidone, spermidine, detergents such as NP-40, Tween 20 and Triton X-100; various proteins that affect secondary structure of DNA including Single Stranded DNA Binding Protein (Amersham Biosciences) and Gene 32 Protein (Amersham Biosciences).

Different kinds of nucleotide degrading enzymes may be used to degrade unincorporated nucleotides and thus reduce the risk of frame shift in sequencing-by-synthesis, such nucleotide degrading enzymes can be, shrimp alkaline phosphates, PPi-ase or apyrase, which are available from Amersham Biosciences and/or Sigma.

The PCR products may be modified in order to improve extension of the sequencing primer in regions of complex secondary structure e.g. incorporation of nucleotide analogues such as dITP, deaza-dGTP, or deaza-dATP.

Examples of fluorophores that may be used as labels for detecting incorporated nucleotides include Alexa Fluor, fluorescein, BODIPY, Rhodamine Green, tetramethylrhodamine, Texas Red, Cascade Blue or Oregon Blue available from Molecular Probes Inc., USA, but may also include molecules such as, cyanine dyes, ROX, TAMRA, R110, R6G, Joe, HEX, or TET.

In addition, fluorescein or another fluorophore could be included on the primer to normalize signals from the incorporated, labeled base where a variation in the amount of template is suspected.

The nucleic acid sequencing template may be prepared by some suitable method, e.g. amplification by PCR from genomic DNA. The template may preferably be modified in such a way as to facilitate immobilization of one (or both) strands on a carrier e.g. through the use of a biotinylated PCR primer to enable immobilization on a streptavidin surface. A number of different means to attach the template to a carrier are available and include coupling the template via specific binding to a hydrophobic compound, an oligonucleotide, an antibody or fragment thereof, a protein, a peptide, an intercalating agent, biotin, or streptavidin or avidin. Furthermore, it can be covalently coupled by using an amino-linker and an epoxy treated carrier.

The conditions for attaching the primer to the template vary depending on the length and sequence of the primer. It is well within the knowledge of a person skilled in the art to establish such conditions. Further guidance may be found in e.g. Maniatis, Molecular Cloning- A Laboratory Manual, Cold Spring Harbor Laboratory Press.

The carrier may be any kind of carrier used within the fields of biotechnology or medicine. Different examples of gels are acrylamide or agarose; examples of beads are solid beads, which can contain a label or a magnetic compound; beads can also be porous, such as Sepharose beads; a surface can be the surface of glass, a plastic polymer, silica or a ceramic material; these surfaces can be used to prepare so-called arrays. One practical example of a plastic surface is the bottom or lower part of a microtiterplate well; even the primer-template complex can form an array on said bottom. A fiber can be a starch fiber or an optical fiber and even the end of a fiber.

Furthermore, the carrier as such can be handled in different ways, gel can cover a surface, a bead can be captured in a channel of a microfluidic device as described in WO 0185341, beads can captured on the surface of a so-called bead array, beads can be dye-labeled and sorted at the same time as the sequencing-by-synthesis reaction is performed.

The template may be immobilized on the carrier and denatured to generate an immobilized single-stranded template to which a sequencing primer may be annealed.

Alternatively, a single-stranded template with unmodified ends could be generated that could be hybridized to an oligonucleotide (sequencing primer) that is itself immobilized on the surface of a carrier via its 5'-end (either before or after hybridization to the template).

The primer is extended by a DNA polymerase in the presence of one or more labeled nucleotides, A, C, G, T or U (or I, inosine). The use of a single labeled nucleotide is preferred. A number of suitable polymerases may be used; it should lack 3'-5' exonuclease activity and accept deoxynucleotides that have been modified with fluorescent molecules. A preferred polymerase is Klenow exo⁻ DNA polymerase. Additional examples of polymerases are defined above. The conditions for performing the extension are easily established by a person skilled in the art, for further guidance, see e.g. Maniatis et al., or the instructions from the supplier of the specific polymerase in use. Basically, a mixture of a DNA polymerase, required buffer, and one of four modified deoxynucleoside triphosphates is added to the primed template and incubated for a suitable length of time to allow the extension to proceed. The modified deoxynucleoside triphosphates may consist of deoxynucleoside triphosphates to which a fluorophore or other label is attached by a cleavable linker arm. The deoxynucleoside triphosphates may comprise, for example, one of the following: fluorophore-SS-dATP, fluorophore-SS-dCTP, fluorophore-SS-dGTP, fluorophore-SS-dUTP or fluorophore-SS-dITP. The fluorophore may or may not be the same for each deoxynucleoside triphosphate. The fluorophore is preferably Cy-5 US 5268486, but the use of other fluorophores, such as Cy-3 or fluorescein, may be envisioned.

The labeled nucleotides may be used singly and sequentially in order to add that nucleotide to the primer. If the nucleotide is complementary to the next nucleotide in the template, the nucleotide will be added to the end of the primer or to the newly synthesized DNA strand. This means that for every single nucleotide in the template, 1-4 nucleotides may have to be tested for incorporation into the DNA strand depending on the knowledge of the sequence.

One or more modified deoxynucleoside triphosphates may be added to the reaction. Excess reagents are removed by washing and the level of fluorescence from the template/primer complex is measured.

Moreover, chain terminating nucleotides, in which blocking groups may be attached at the 3' moiety of the deoxyribose group of the labeled nucleotide to prevent non-specific incorporation, can be used. The attachment of the label on the nucleotide may advantageously be on the 3' moiety of the deoxyribose group. The label may then be removed to generate a 3' -OH group to allow subsequent elongation of the chain. Preferably, the attachment of the label to the nucleotide is by means of a chemically or enzymatically cleavable linker. In the case where a disulfide bridge is between the 3'-end of the sugar and a label the same problems as described above will be envisaged, which the present invention will also solve.

The detection of incorporated nucleotide depends on the type of label being used. For fluorescent labels, the detection may be based on different detection methods, including laser excitation combined with the use of a CCD camera, photomultiplier, or photon counter. The detection of the label may be performed with the label still attached to the nucleotide, or it may be performed after cleavage of the link between nucleotide and label. The advantage with the label still attached to the primer/template complex is that the signal will be localized and support an array based concept.

The label attached to the incorporated nucleotide may or may not be removed or neutralized. Various ways to remove the label can be envisioned, depending on the nature of the link between the label and the nucleotide. In the case where the link consists of a disulfide bridge, the link may be broken by reducing the thiol groups. This may be accomplished by the use of a reducing agent such as DTT (dithiothreitol) or TCEP (tris-(2-carboxyethyl)phosphine hydrochloride). Other reducing agents may be employed. Various ways to neutralize the emitted light from a label are e.g. by bleaching, preferably photo-bleaching or by adding a compound that interacts with the label in such a way that it makes the label undetectable; such compounds can be other labels, which will reduce the emitted light by quenching.

It is envisioned that the method described herein may be applicable to a wide range of fields, such as genotyping by scoring SNPs in microfabricated devices or on arrays of primers or templates immobilized on plastic, silicon or glass. A further application would be the de-novo sequence determination of templates on similar devices or sequence determination of templates of known sequence on similar devices. A further application is described in WO99/35293A2, which describes solid phase bead selection of differentially expressed genes. Another application of the present invention is to use it in an apparatus and system for simultaneous analysis of analytes anchored to microparticles. Such a system is disclosed in WO98/53300.

### EXAMPLES

### Example 1:

### Capping thiol groups for protecting neighboring Cy5-SS-dCTP incorporated from premature cleavage.

The oligonucleotide templates E3PN10b and E3PN22b, biotinylated at their 5' ends were annealed to a primer, NUSPT-Fluorescein as shown. The bases to be incorporated are indicated in bold with the two positions for C incorporations indicated in **underlined bold.**

E3PN10b

Fluorescein-GTAAAACGACGGCCAGTCTGAAC
CAACATTTTGCTGCCGGTCAGACTTGCTTAAGGTCG-biotin

E3PN22b

Fluorescein-GTAAAACGACGGCCAGTCTC
CAACATTTTGCTGCCGGTCAGAGCTTAAGGTCG-biotin

The annealing reaction consisted of the following steps: 50 pmole template were mixed with 30 pmole primer in a total of 250 µL Annealing Buffer (20 mM Tris-acetate, 5 mM MgAc₂, pH 7.6) (also termed AB), incubated at 80 °C for 5' and allowed to cool. The annealed oligonucleotides were then immobilized on Streptavidin Sepharose High Performance beads (Amersham Biosciences) by incubation with 250 µL of bead slurry and 500 µL Binding Buffer (10 mM Tris-HCl, 2 M NaCl, 1 mM EDTA, 0.1% Tween 20) (also termed BB) for 20 minutes with shaking. Excess oligonucleotides were removed by washing the beads in filter tubes (Nanosep MF GHP 0.45 µm, Pall) and the beads were resuspended in 500 µL 2xAB (40 mM Tris-Acetate, 10 mM MgAc₂, pH 7.6) and distributed in 50 µL aliquots into wells in a filter plate (Multiscreen, Millipore).

The first Cy5-SS-dCTP was incorporated by adding a reaction mixture (1 µM Cy5-SS-dCTP, 5U Klenow exo- DNA Polymerase in 2xAB buffer) and incubating for 2 minutes at 37 °C. The beads were washed 4x with 200 µL TENT buffer (40 mM Tris-HCl, 50 mM NaCl, 1 mM EDTA, 0.1% Tween 20, pH 8.8) by sucking the buffer through a filter under vacuum, resuspended in 50 µL TENT-buffer and transferred to fluorimeter plates to measure the fluorescence of the Cy5-labelled nucleotide (excitation 590 nm, emission 670 nm) and the fluorescence of the fluorescein-labeled primer (excitation 485nm, emission 535 nm) using a fluorimeter (Victor2, Perkin-Elmer). The fluorescein signal was used to normalize results for variation in transfer of beads.

The contents of the wells were pooled in groups of three and transferred to filter tubes. The Cy5 label on the labeled nucleotide was removed by incubating with Cleavage Buffer (50 mM dithiothreitol, 50 mM NaCl, 40 mM Tris-HCl, 20 mM MgCl₂, pH 8.4) (also termed CB) for 5 minutes at room temperature and the beads were washed 3 times with 400 µL TENT Buffer followed by 2 times with 400 µL 2xAB. The contents of the tubes were incubated with 100 µL (1) 2xAB as control, (2) 1 mM iodoacetamide (Sigma I-1149) or (3) 10 mM iodoacetamide in 2xAB, for 5 minutes at room temperature protected from light. Excess iodoacetamide was then inactivated by adding 100 µL Cleavage Buffer, followed by washing 4 times with 400 µL 2xAB.

The beads were resuspended in 2xAB and redistributed between wells in a filter plate. Natural nucleotides were incorporated to further extend the primer to the next position for incorporation of a C (E3PN10b: 1 µM each of dATP, dGTP and dTTP, 5U Klenow exo- DNA Polymerase in 2xAB buffer; E3PN22b: as for E3PN10b but with dTTP only). The reaction was carried out as above and the beads were washed in AB.

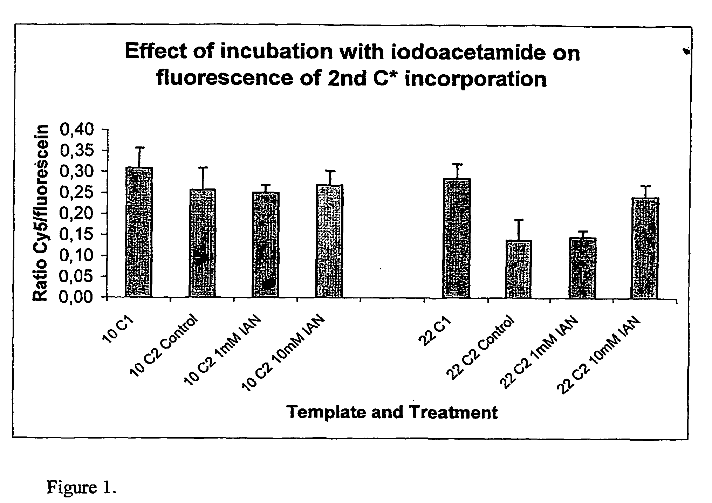

The second Cy5-SS-dCTP was then incorporated in the same way as the first. Fluorescence was again measured to determine the incorporation of the second label. The results are shown in Figure 1. The results indicate that the fluorescent signal from the first and second incorporations of Cy5-SS-dCTP were similar for E3PN10b/NUSPT, with or without treatment with iodoacetamide, whereas the signal from the second incorporation of Cy5-SS-dCTP into E3PN22b/NUSPT was lower than that from the first incorporation in controls, as observed in previous experiments. The signal was restored when, after the first incorporation of Cy5-SS-dCTP followed by cleavage, the primer/template complex was treated with 10 mM iodoacetamide.. This indicates that capping the exposed thiol group protected the second, neighboring Cy5-SS-dCTP from premature cleavage.

### Example 2:

### Achieving a linear relationship between fluorescence signal and number of bases incorporated in homopolymer stretches with Cy5-SS-dCTP/dCTP mixes

E3PN4B

Fluorescein-GTAAAACGACGGCCAGTAC
CAACATTTTGCTGCCGGTCATGCTGCTTAAGGTCG-biotin

E3PN5B

Fluorescein-GTAAAACGACGGCCAGTACC
CAACATTTTGCTGCCGGTCATGGCTGCTTAAGGTCG-biotin

E3PN6B

Fluorescein-GTAAAACGACGGCCAGTACCC
CAACATTTTGCTGCCGGTCATGGGCTGCTTAAGGTCG-biotin

E3PN28B

Fluorescein-GTAAAACGACGGCCAGTACCCC
CAACATTTTGCTGCCGGTCATGGGGCTGCTTAAGGTCG-biotin

Oligonucleotide templates, biotinylated at their 5' ends were annealed to a primer, NUSPT-Fluorescein as shown. The bases to be incorporated are indicated in **bold** with the positions for C incorporations indicated in **underlined bold****.**

The annealing reaction consisted of the following steps: 50 pmole template were mixed with 30 pmole primer in a total of 250 µL Annealing Buffer (20 mM Tris-acetate, 5 mM MgAc₂, pH 7.6), incubated at 80 °C for 5 minutes and allowed to cool. The annealed oligonucleotides were then immobilized on Streptavidin Sepharose High Performance beads (Amersham Biosciences) by incubation with 50 µL of bead slurry and 500 µL Binding Buffer (10 mM Tris-HCl, 2 M NaCl, 1 mM EDTA, 0.1% Tween 20) for 20 minutes with shaking. Excess oligonucleotides were removed by washing the beads in filter tubes (Nanosep MF GHP 0.45 µm, Pall) and the beads were resuspended in 500 µL 2xAB (40 mM Tris-Acetate, 10 mM MgAc₂, pH 7.6) and distributed in 50 µL aliquots into wells in a filter plate (Multiscreen, Millipore).

The first dATP and Cy5-SS-dCTP were incorporated by adding a reaction mixture (1µM dATP, 1µM C-mix*, 5U Klenow exo⁻ Polymerase in 2xAB buffer) and incubating for 2' at 37 °C. *C-mix was prepared using equal volumes of 100% Cy5-dCTP, 100% Cy5-SS-dCTP, or 20% Cy5-SS-dCTP + 80% dCTP. The beads were washed 4x with 400 µL TENT buffer (40 mM Tris-HCl, 50 mM NaCl, 1 mM EDTA, 0.1% Tween 20, pH 8.8) by sucking the buffer through a filter under vacuum, resuspended in 50 µL TENT and transferred to fluorimeter plates to measure the fluorescence of the Cy5-labelled nucleotide (excitation 590 nm, emission 670 nm) and the fluorescence of the fluorescein-labeled primer (excitation 485nm, emission 535 nm) using a fluorimeter (Victor2, Perkin-Elmer). The fluorescein signal was used to normalize results for variation in transfer of beads.

The level of incorporation of nucleotides was checked by analyzing the immobilized templates by pyrosequencing using PSQ 96 and associated kits according to the manufacturers instructions (Pyrosequencing AB, Sweden) such that the absence of a peak at the point of dispensing C was indication of complete incorporation in the foregoing experiment.

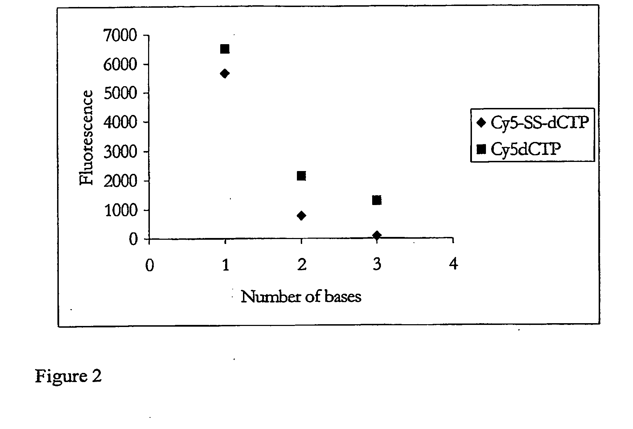

The result in Figure 2 shows a significant reduction in fluorescence signal when 100% labeled dCTP was used (either Cy5-dCTP or Cy5-SS-dCTP). This indicates strong quenching of the signal due to the proximity of Cy5 groups on the extended primer.

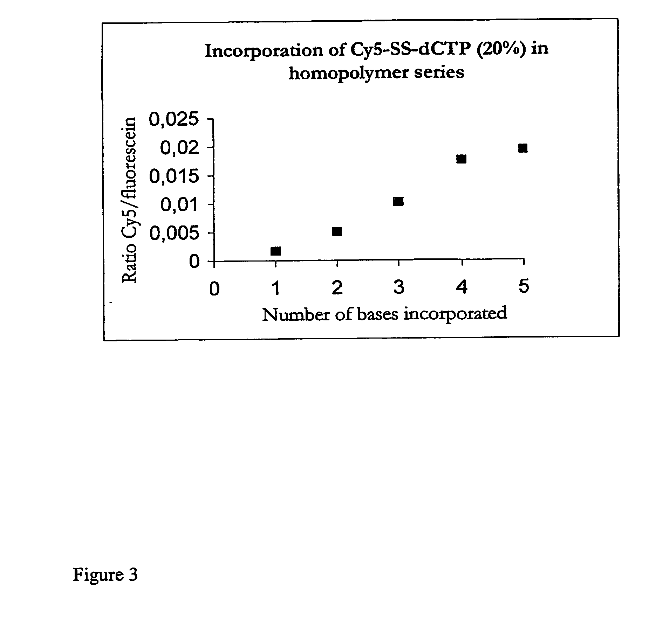

The result in Figure 3 shows linearity in the signal when incorporations were performed with a mixture of 20% Cy5-SS-dCTP and 80% dCTP.

All incubations gave better than 95% incorporation as assessed by pyrosequencing (results not shown).

### Example 3

### Example: Sequencing, using "directed dispensation", of the oligonucleotide E3PN19b

NUSPT: fluorescein-GTAAAACGACGGCCAGTUCAGACGAA
E3PN19b        CAACATTTTGCTGCCGGTCAAGTCTGCTTAAGGTCG-
biotin

Five pmole of template E3PN19b and 3 pmole primer NUSPT-FL were annealed at 80°C for five minutes in 25 µl Annealing Buffer (20 mM Tris-acetate, 5 mM MgAc₂, pH 7.6). After cooling to room temperature, the template was bound to streptavidin beads by adding 4 µl bead slurry (Streptavidin Sepharose High Performance beads) together with 29 µl Binding buffer (10 mM Tris-HCl, 2 M NaCl, 1 mM EDTA, 0.1% Tween-20) followed by incubation at room temperature for 20 min with shaking at 1400 rpm.

The beads were transferred to a filter plate (Multiscreen, Millipore) and washed four times with 2xAB (40 mM Tris-acetate, 10 mM MgAc₂, pH 7.6). The filter plate was pre-warmed at 37 °C for 2 minutes. The first base was incorporated by adding 50 µL Reaction Mixture (0.5 µM Cy5-SS-dUTP, 0.5 µM dUTP, 5 U Klenow exo⁻, 2xAB) and incubating at 37 °C for 2 minutes.

The wells of the filter plate were washed four times with TENT (40 mM Tris-HCl pH 8.8, 50 mM NaCl, **1** mM EDTA, 0.1% Tween 20) by sucking the buffer through a filter under vacuum. The beads were resuspended in 50 µl TENT and transferred to a fluorimeter plate to a fluorimeter plate to measure the fluorescence of the Cy5-labelled nucleotide (excitation 590 nm, emission 670 nm) and the fluorescence of the fluorescein-labeled primer (excitation 485nm, emission 535 nm) using a fluorimeter (Victor2, Perkin-Elmer). The fluorescein signal was used to normalize results for variation in transfer of beads. After measuring, the beads were transferred back into the filter plate and the Cy5-label was cleaved from the incorporated dUTP by incubation with Cleavage Buffer (250 mM dithiothreitol, 50 mM NaCl, 40 mM Tris-HCl, 20 mM MgCl₂, pH 8.4) for 3 minutes at 37°C. The filter plate was then washed two times in TENT and two times in 2xAB.

Subsequent Cy5-SS-dNTPs were incorporated in the same manner as the first and cleaved as described above. The sequencing reaction mixes were the same for all four deoxynucleotides except for the proportion of labeled dNTPs.

The mixes contained 20% Cy5-SS-dCTP, 30% Cy5-SS-dATP or 30% Cy5-SS-dGTP with the balance made up with the corresponding natural deoxynucleotide.

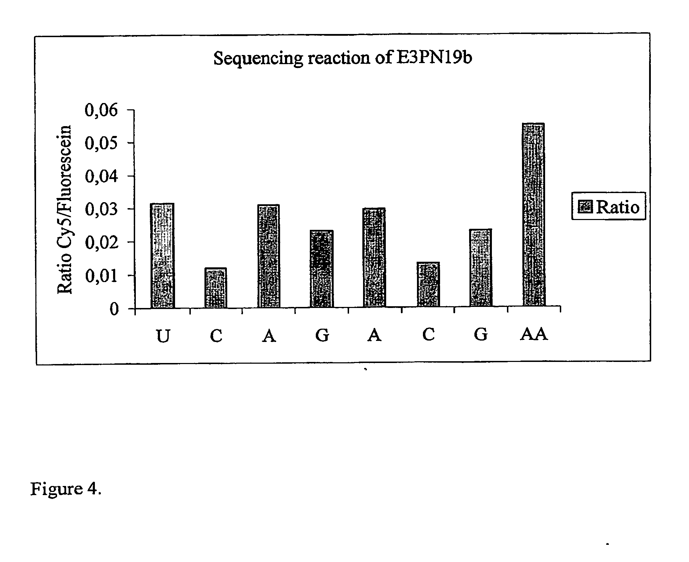

As can be seen in Figure 4, the signals obtained were reproducible and stable throughout the sequence for the different nucleotides. The internal variation in signal height between different bases was due to differences in the way Klenow exo⁻ polymerase accepts the labeled nucleotides. The level of incorporation of nucleotides was checked by analyzing the immobilized templates by pyrosequencing using PSQ 96 and associated kits according to the manufacturers instructions (Pyrosequencing AB, Sweden) such that the absence of a peak at the point of dispensing respective dNTPs was indication of complete incorpotanon in the foregoing experiment. AU incubations gave better than 95% incorporation as assessed by pyrosequencing (results not shown).

### Example 4: Determining the selectivity of Klenow exo- DNA polymerase for labeled/unlabelled nucleotides

E3PN10B

Fluorescein-GTAAAACGACGGCCAGTC
CAACATTTTGCTGCCGGTCAGACTTGCTTAAGGTCG-biotin

E3PN4B

Fluorescein-GTAAAACGACGGCCAGTA

CAACATTTTGCTGCCGGTCATGCTGCTTAAGGTCG-biotin

E3PN12b

Fluorescein-GTAAAACGACGGCCAGTG
CAACATTTTGCTGCCGGTCACTACTGCTTAAGGTCG-biotin

E3PN18b

Fluorescein-GTAAAACGACGGCCAGTU

CAACATTTTGCTGCCGGTCAACGCTGCTTAAGGTCG-biotin

Oligonucleotide templates, biotinylated at their 5' ends were annealed to a primer, NUSPT-Fluorescein as shown. The bases to be incorporated are indicated in underlined bold.

The annealing reaction consisted of the following steps for each replicate : 5 pmole template were mixed with 3 pmole primer in a total of 25 µL Annealing Buffer (20 mM Tris-acetate, 5 mM MgAc₂, pH 7.6), incubated at 80 °C for 5 minutes and allowed to cool. The annealed oligonucleotides were then immobilized on Streptavidin Sepharose High Performance beads (Amersham Biosciences) by incubation with 25 µL of bead slurry and 50 µL Binding Buffer (10 mM Tris-HCl, 2 M NaCl, 1 mM EDTA, 0.1 % Tween 20) for 20 minutes with shaking. Excess oligonucleotides were removed by washing the beads in 2xAB (40 mM Tris-Acetate, 10 mM MgAc₂, pH 7.6) in filter tubes (Nanosep MF GHP 0.45 µm, Pall) and subsequently resuspended in 50 µL 2xAB and transferred to wells in a filter plate (Multiscreen, Millipore).

The relevant Cy5-SS-dNTP was incorporated into the first position by adding a reaction mixture (1 µM dNTP-mix*, 5U Klenow exo- Polymerase in 2xAB buffer) and incubating for 2 minutes at 37 °C. *dNTP-mix was prepared using 100, 80, 50, 20 or 10% Cy5-SS-dNTP made up to 100% with the corresponding non-labeled dNTP. The beads were washed 4x with 200 µL TENT buffer (40 mM Tris-HCl, 50 mM NaCl, 1 mM EDTA, 0.1 % Tween 20, pH 8.8) by sucking the buffer through a filter under vacuum, resuspended in 50 µL TENT and transferred to fluorimeter plates to measure the fluorescence of the Cy5-labelled nucleotide (excitation 590 nm, emission 670 nm) and the fluorescence ofthe fluorescein-labeled primer (excitation 485nm, emission 535 nm) using a fluorimeter (Victor2, Perkin-Elmer). The fluorescein signal was used to normalize results for variation in transfer of beads.

The level of incorporation of nucleotides was checked by analyzing the immobilized templates by pyrosequencing using PSQ 96 and associated kits according to the manufacturers instructions (Pyrosequencing AB, Sweden) such that the absence of a peak at the point of dispensing the relevant nucleotide was indication of complete incorporation in the foregoing experiment. All incubations gave better than 95% incorporation as assessed by pyrosequencing (results not shown).

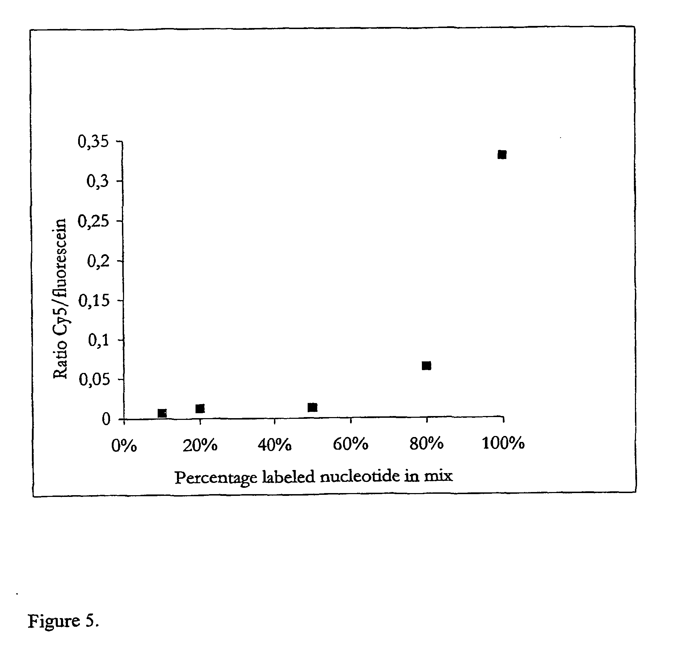

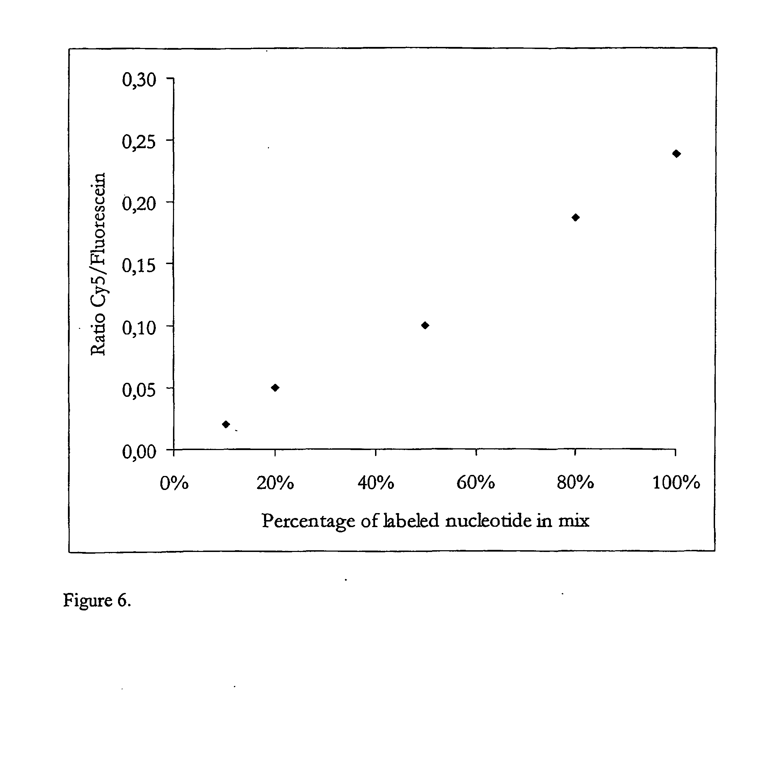

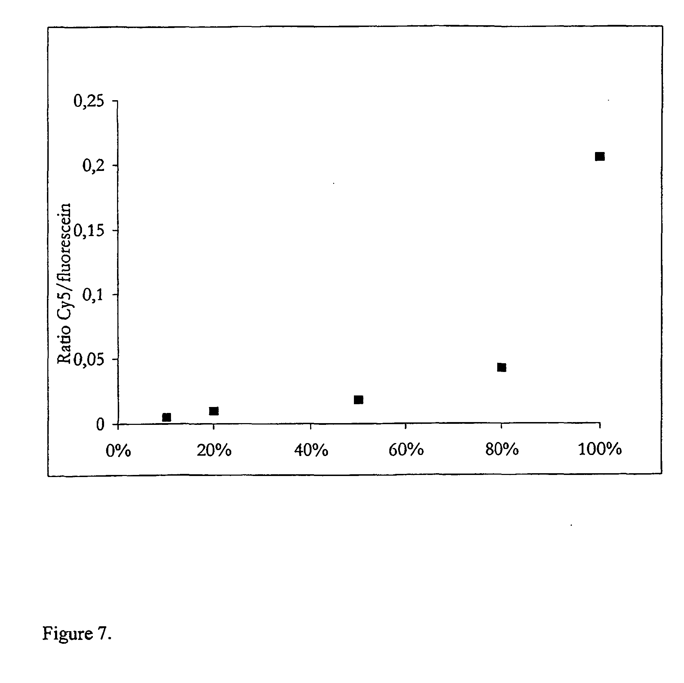

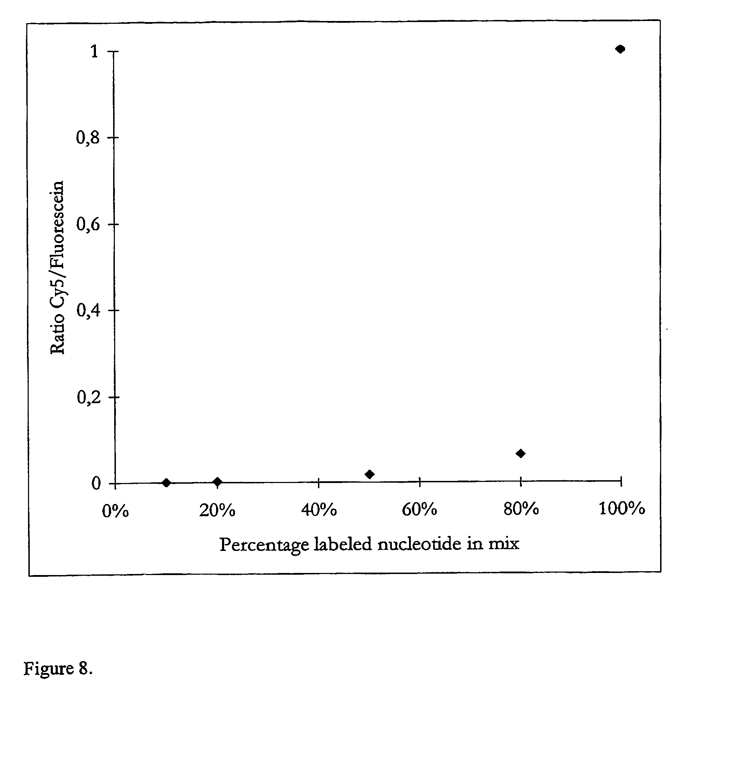

The results in Figures 5-8 show the selectivity of the polymerase for labeled against non-labeled nucleotides. There are clear differences in how the polymerase accepts the different Cy5-SS-nucleotides, in particular between U* and G*.

### Example 5: Determining the relationship between fluorescence signal and number of bases incorporated in homopolymer stretches with Cy5-SS-dGTP/dGTP mixes according to WO 00/53812

E3PN13b: 5'-GCTGGAATTCGTATGCACTGGCCGTCGTTTTACAAC-3'

E3PN24b: 5′-GCTGGAATTCGTATGCCACTGGCCGTCGTTTTACAAC-3′

E3PN25b: 5'-GCTGGAATTCGTATGCCCACTGGCCGTCGTTTTACAAC-3'

NUSPT-FL: 5'-Fluorescein-GTA AAA CGA CGG CCA GT-3'

Five pmole of template and 3 pmole primer NUSPT-FL were annealed at 80°C for five minutes in 25 µl Annealing Buffer (20 mM Tris-acetate, 5 mM MgAc₂, pH 7.6). After cooling to room temperature, the template was bound to streptavidin beads by adding 4 µl bead slurry (Streptavidin Sepharose High Performance beads) together with 29 µl Binding buffer (10 mM Tris-HCl, 2 M NaCl, 1 mM EDTA, 0.1 % Tween-20) followed by incubation at room temperature for 20 min with shaking at 1400 rpm.

The beads were transferred to a filter plate (Multiscreen, Millipore) and washed four times with 2xAB (40 mM Tris-acetate, 10 mM MgAc₂, pH 7.6).

One, two or three Cy5-SS-dGTPs were incorporated by adding 30 µL Reaction Mixture (0.2 µM Cy5-SS-dGTP, 0.1 µM dGTP, 6.5 U Sequenase version 2.0, 40 mM Tris-HCl pH 7.5, 20 mM MgCl₂, 50 mM NaCl) and incubating at room temperature for 4 minutes.

The wells of the filter plate were washed four times with TENT (40 mM Tris-HCl pH 8.8, 50 mM NaCl, 1 mM EDTA, 0.1% Tween 20) under vacuum. The beads were resuspended in 50 µl TENT and transferred to a fluorimeter plate. The fluorescent signal from fluorescein on the primer and Cy5 on the incorporated dNTP was measured using a fluorimeter (VICTOR2, PerkinElmer). The fluorescein signal was used to normalise variations in the amount of beads transferred to the fluorimeter plate.

## Claims

1. A method for determining the sequence of a nucleic acid molecule comprising the steps of;
a) providing a single-stranded form of said nucleic acid molecule;
b) hybridizing a primer to said single stranded form of said nucleic acid molecule to form a template/primer complex;
c) enzymatically extending the primer by the addition of a polymerase and a mixture of at least one nucleotide and at least one labeled derivative of the at least one nucleotide, wherein the at least one labeled derivative of the at least one nucleotide comprises a label linked to the nucleotide via a cleavable link and wherein the amount of labeled derivative of the at least one nucleotide in said mixture of the at least one nucleotide and the labeled derivative of the at least one nucleotide is within the range of 1-50 mole-%, 1-40 mole-%, 1-30 mole-%, or 1-20 mole-%.
d) determining the type of nucleotide added to the primer, and
e) repeating steps c) to d) at least once.

2. A method according to claim 1, in which the amount of labelled derivative of the at least one nucleotide in said mixture is within the range of 5-50 mole-%, 5-40 mole-%, 5-30 mole-%, or 5-20 mole-%.

3. A method according to claim 1, in which the amount of labelled derivative of the at least one nucleotide in said mixture is within the range of 10-50 mole-%, 10-40 mole-%, 10-30 mole-%, or 10-20 mole-%.

4. A method according to any one of claims 1 - 3, wherein the single-stranded form of said nucleic acid molecule is attached to a carrier.

5. A method according to claim 4, wherein the the means for attachment is selected from the group of: a) specific binding to a hydrophobic compound, an oligonucleotide, an antibody or a fragment thereof, a protein, a peptide, an intercalating agent, biotin, streptavidin or avidin; or b) covalent coupling using an amino-linker and an epoxy-treated carrier.

6. A method according to claim 4, wherein the carrier is selected from the group of a gel, a solid or porous bead, a surface or a fiber.

7. A method according to any one of claims 1 - 3, in which the label is neutralized after step d) by the addition of a label-interacting agent or by bleaching.

8. A method according to claim 7, in which the bleaching is performed by photo-bleaching.

9. A method according to claim 1-3 in which the link between the incorporated nucleotide and the label is cleaved after step **d**).

10. A method according to claim 1-3, in which the link between the fluorophore and nucleotide is a disulfide bond.

11. A method according to claim 10 in which the cleavage is performed by the addition of a reducing agent, thereby exposing a thiol group.

12. A method according to claims 10 or 11, in which the exposed thiol group is capped by a suitable reagent, such as iodoacetamide or N-ethylmaleimide.

13. A method according to any of the claims above in which the linker between the disulfide bridge and the base is shorter than 8 atoms.

14. A method according to any ofthe above claims in which the step c) is performed at a pH below 7, preferably at a pH below 6.5, or more preferably at a pH below 6.

15. A method according to any of the above claims, in which the derivative of said nucleotide is a dideoxynucleotide or an acyclic nucleotide analog.

16. A method according to any of the above claims, in which an agent chosen from the group comprising the following; alkaline phosphatase, PPi-ase, apyrase, dimethylsulfoxide, polyethylene glycol, polyvinylpyrollidone, spermidine, detergents such as NP-40, Tween 20 and Triton X-100; various proteins that affect secondary structure of DNA including Single Stranded DNA Binding Protein (SSB) or the protein of Gene 32, is added.

17. A kit comprising, in separate compartments, a mixture of at least one nucleotide and at least one labelled derivative of the at least one nucleotide, wherein the at least one labeled derivative of the at least one nucleotide comprises a label linked to the nucleotide via a cleavable link and wherein the amount of labeled derivative of the at least one nucleotide in said mixture of the at least one nucleotide and the labeled derivative of the at least one nucleotide is within the range of 1-50 mole-%, 1-40 mole-%, 1-30 mole-%, or 1-20 mole-%, preferably in the range of 5-20 mole-%, 5-30 mole-%, 5-40 mole-% or 5-50 mole-%, and even more preferably in the range of 10-20 mole-%, 10-30 mole-%, 10-40 mole-% or 10-50 mole-%, and a reducing agent.

18. A kit according to claim 17 further comprising at least one ofthe following components; a DNA polymerase, a carrier, a capping agent, an apyrase, an alkaline phosphatase, a PPi-ase, a single strand binding protein or the protein of Gene 32, for performing the method according to any of the claims 1-14.

## Patentansprüche

1. Verfahren zur Bestimmung der Sequenz eines Nukleinsäuremoleküls, umfassend die folgenden Schritte:
a) Bereitstellung einer Einzelstrang-Form des Nukleinsäuremoleküls,
b) Hybridisieren eines Primers an die Einzelstrang-Form des Nukleinsäuremoleküls zur Bildung eines Matrizen/Primer-Komplexes,
c) enzymatische Verlängerung des Primers durch Addition einer Polymerase oder einer Mischung von mindestens einem Nukleotid und mindestens einem markierten Derivat des mindestens einen Nukleotids, wobei das mindestens eine markierte Derivat des mindestens einen Nukleotids eine Markierung umfaßt, gebunden an das Nukleotid über eine spaltbare Bindung und wobei die Menge des markierten Derivats des mindestens einen Nukleotids in der Mischung des mindestens einen Nukleotids und des markierten Derivats des mindestens einen Nukleotids im Bereich von 1 bis 50 mol%, 1 bis 40 mol%, 1 bis 30 mol% oder 1 bis 20 mol% liegt,
d) Bestimmung der Art des zu dem Primer zugefügten Nukleotids und
e) mindestens einmalige Wiederholung der Schritte c) bis d).

2. Verfahren gemäß Anspruch 1, wobei die Menge des markierten Derivats des mindestens einen Nukleotids in der Mischung im Bereich von 5 bis 50 mol%, 5 bis 40 mol%, 5 bis 30 mol% oder 5 bis 20 mol% liegt.

3. Verfahren gemäß Anspruch 1, wobei die Menge des markierten Derivats des mindestens einen Nukleotids in der Mischung im Bereich von 10 bis 50 mol%, 10 bis 40 mol%, 10 bis 30 mol% oder 10 bis 20 mol% liegt.

4. Verfahren gemäß einem der Ansprüche 1 bis 3, wobei die Einzelstrang-Form des Nukleinsäuremoleküls an einen Träger angebunden ist.

5. Verfahren gemäß Anspruch 4, wobei das Mittel zur Anbindung ausgewählt ist aus der Gruppe: a) spezifische Bindung an eine hydrophobe Verbindung, ein Oligonukleotid, einen Antikörper oder ein Fragment davon, ein Protein, ein Peptid, ein interkalierendes Mittel, Biotin, Streptavidin oder Avidin, oder b) kovalente Kopplung unter Verwendung eines Aminolinkers und eines epoxybehandelten Trägers.

6. Verfahren gemäß Anspruch 4, wobei der Träger ausgewählt ist aus der Gruppe eines Gels, eines festen oder porösen Kügelchens, einer Oberfläche oder einer Faser.

7. Verfahren gemäß einem der Ansprüche 1 bis 3, wobei die Markierung nach Schritt d) durch die Zugabe eines mit der Markierung interagierenden Mittels oder durch Bleichen neutralisiert wird.

8. Verfahren gemäß Anspruch 7, wobei das Bleichen durch Fotobleichen durchgeführt wird.

9. Verfahren gemäß einem der Ansprüche 1 bis 3, wobei die Bindung zwischen dem eingebauten Nukleotid und der Markierung nach Schritt d) gespalten wird.

10. Verfahren gemäß einem der Ansprüche 1 bis 3, wobei die Bindung zwischen dem Fluorophor und dem Nukleotid eine Disulfidbindung ist.

11. Verfahren gemäß Anspruch 10, wobei die Spaltung durch Zugabe eines Reduktionsmittels durchgeführt wird, wodurch eine Thiolgruppe exponiert wird.

12. Verfahren gemäß Anspruch 10 oder 11, wobei die exponierte Thiolgruppe durch ein geeignetes Reagens, wie z.B. Iodacetamid oder N-Ethylmaleimid, mit einer Kappe versehen wird.

13. Verfahren gemäß einem der obigen Ansprüche, wobei der Linker zwischen der Disulfidbrücke und der Base kürzer als 8 Atome ist.

14. Verfahren gemäß einem der obigen Ansprüche, wobei Schritt c) bei einem pH unterhalb von 7, vorzugsweise einem pH unterhalb von 6,5 oder noch bevorzugter bei einem pH unterhalb von 6, durchgeführt wird.

15. Verfahren gemäß einem der obigen Ansprüche, wobei das Derivat des Nukleotids ein Dideoxynukleotid oder ein acyclisches Nukleotidanalog ist.

16. Verfahren gemäß einem der obigen Ansprüche, wobei ein Mittel, ausgewählt aus der Gruppe umfassend die folgenden: alkalische Phosphatase, PPi-ase, Apyrase, Dimethylsulfoxid, Polyethylenglycol, Polyvinylpyrrolidon, Spermidin, Detergenzien wie NP-40, Tween 20 und Triton X-100; verschiedenen Proteinen, die die Sekundärstruktur der DNA beeinflussen, einschließlich Einzelstrang-DNA-Bindungsprotein (SSB) oder dem Protein von Gen 32 zugefügt wird.

17. Kit, umfassend in getrennten Abteilen eine Mischung von mindestens einem Nukleotid und mindestens einem markierten Derivat des mindestens einen Nukleotids, wobei das mindestens eine markierte Derivat des mindestens einen Nukleotids eine Markierung umfaßt, gebunden an das Nukleotid über eine spaltbare Bindung und wobei die Menge des markierten Derivats des mindestens einen Nukleotids in der Mischung des mindestens einen Nukleotids und des markierten Derivats des mindestens einen Nukleotids im Bereich von 1 bis 50 mol%, 1 bis 40 mol%, 1 bis 30 mol% oder 1 bis 20 mol% liegt, vorzugsweise im Bereich von 5 bis 20 mol%, 5 bis 30 mol%, 5 bis 40 mol% oder 5 bis 50 mol% und noch bevorzugter im Bereich von 10 bis 20 mol%, 10 bis 30 mol%, 10 bis 40 mol% oder 10 bis 50 mol%, und ein Reduktionsmittel.

18. Kit gemäß Anspruch 17, weiterhin umfassend mindestens einen der folgenden Bestandteile: eine DNA-Polymerase, einen Träger, ein kappenbildendes Mittel, eine Apyrase, eine alkalische Phosphatase, eine PPi-ase, ein Einzelstrang-Bindungsprotein oder das Protein von Gen 32 zur Durchführung des Verfahrens gemäß einem der Ansprüche 1 bis 14.

## Revendications

1. Procédé de détermination de la séquence d'une molécule d'acide nucléique comprenant les étapes de :
a) fourniture d'une forme simple brin de ladite molécule d'acide nucléique ;
b) hybridation d'une amorce à ladite forme simple brin de ladite molécule d'acide nucléique pour former un complexe matrice/amorce ;
c) extension par voie enzymatique de l'amorce par addition d'une polymérase et d'un mélange d'au moins un nucléotide et au moins un dérivé marqué du au moins un nucléotide, dans lequel le au moins un dérivé marqué du au moins un nucléotide comprend un marqueur lié au nucléotide par l'intermédiaire d'une liaison clivable et dans lequel la quantité de dérivé marqué du au moins un nucléotide dans ledit mélange du au moins un nucléotide et du dérivé marqué du au moins un nucléotide est comprise dans la gamme de 1 à 50% en moles, 1 à 40% en moles, 1 à 30% en moles, ou 1 à 20% en moles ;
d) détermination du type de nucléotide ajouté à l'amorce ; et
e) répétition des étapes c) à d) au moins une fois.

2. Procédé selon la revendication 1, dans lequel la quantité de dérivé marqué du au moins un nucléotide dans ledit mélange est comprise dans la gamme de 5 à 50% en moles, 5 à 40% en moles, 5 à 30% en moles, ou 5 à 20% en moles.

3. Procédé selon la revendication 1, dans lequel la quantité de dérivé marqué du au moins un nucléotide dans ledit mélange est comprise dans la gamme de 10 à 50% en moles, 10 à 40% en moles, 10 à 30% en moles, ou 10 à 20% en moles.

4. Procédé selon l'une quelconque des revendications 1 à 3, dans lequel la forme simple brin de ladite molécule d'acide nucléique est liée à un support.

5. Procédé selon la revendication 4, dans lequel le moyen de liaison est choisi dans le groupe consistant en : a) liaison spécifique à un composé hydrophobe, un oligonucléotide, un anticorps ou un fragment de celui-ci, une protéine, un peptide, un agent d'intercalation, de la biotine, de la streptavidine ou de l'avidine ; ou b) couplage covalent en utilisant un élément de liaison amino et un support traité époxy.

6. Procédé selon la revendication 4, dans lequel le support est choisi dans le groupe consistant en un gel, une bille solide ou poreuse, une surface ou une fibre.

7. Procédé selon l'une quelconque des revendications 1 à 3, dans lequel le marqueur est neutralisé après l'étape d) par addition d'un agent d'interaction avec le marqueur ou par décoloration.

8. Procédé selon la revendication 7, dans lequel la décoloration est réalisée par photo-décoloration.

9. Procédé selon les revendications 1 à 3, dans lequel la liaison entre le nucléotide incorporé et le marqueur est clivé après l'étape d).

10. Procédé selon les revendications 1 à 3, dans lequel la liaison entre le fluorophore et le nucléotide est une liaison disulfure.

11. Procédé selon la revendication 10, dans lequel le clivage est réalisé par addition d'un agent de réduction, exposant de ce fait un groupe thiol.

12. Procédé selon les revendications 10 ou 11, dans lequel le groupe thiol exposé est coiffé par un réactif convenable, tel que iodoacétamide ou N-éthylmaléimide.

13. Procédé selon l'une quelconque des revendications ci-dessus, dans lequel l'élément de liaison entre le pont disulfure et la base comporte moins de 8 atomes.

14. Procédé selon l'une quelconque des revendications ci-dessus, dans lequel l'étape c) est réalisée à un pH inférieur à 7, de préférence à un pH inférieur à 6,5, ou de préférence encore à un pH inférieur à 6.

15. Procédé selon l'une quelconque des revendications ci-dessus, dans lequel le dérivé dudit nucléotide est un didésoxynucléotide ou un analogue de nucléotide acyclique.

16. Procédé selon l'une quelconque des revendications ci-dessus, dans lequel un agent choisi dans le groupe comprenant les suivants : phosphatase alcaline, PPi-ase, apyrase, diméthylsulfoxyde, polyéthylène glycol, polyvinylpyrrolidone, spermidine, détergents tels que NP-40, Tween 20 et Triton X-100 ; diverses protéines qui affectent la structure secondaire d'un ADN comprenant une protéine fixatrice d'ADN simple brin (SSB) ou la protéine du Gène 32, est ajouté.

17. Kit comprenant, dans des compartiments séparés, un mélange d'au moins un nucléotide et au moins un dérivé marqué du au moins un nucléotide, dans lequel le au moins un dérivé marqué du au moins un nucléotide comprend un marqueur lié au nucléotide par l'intermédiaire d'une liaison clivable et dans lequel la quantité de dérivé marqué du au moins un nucléotide dans ledit mélange du au moins un nucléotide et du dérivé marqué du au moins un nucléotide est comprise dans la gamme de 1 à 50% en moles, 1 à 40% en moles, 1 à 30% en moles, ou 1 à 20% en moles, de préférence dans la gamme de 5 à 20% en moles, 5 à 30% en moles, 5 à 40% en moles, ou 5 à 50% en moles, et même de préférence encore dans la gamme de 10 à 20% en moles, 10 à 30% en moles, 10 à 40% en moles, ou 10 à 50% en moles, et un agent de réduction.

18. Kit selon la revendication 17 comprenant de plus au moins un des composants suivants : une ADN polymérase, un support, un agent coiffant, une apyrase, une phosphatase alcaline, une PPi-ase, une protéine fixatrice simple brin ou la protéine du Gène 32, pour réaliser le procédé selon l'une quelconque des revendications 1 à 14.
